# EUROPEAN PATENT SPECIFICATION

(11) **EP 3 917 498 B1**
(45) Date of publication and mention of the grant of the patent: **01.01.2025**
(21) Application number: 20713087.3
(22) Date of filing: 29.01.2020
(51) Int. Cl.: A61K 9/107, A61K 47/14, A61K 47/26, A61K 38/14, A61K 31/546, A61K 31/375, A61K 47/44

(54) **SELF-EMULSIFYING PHARMACEUTICAL COMPOSITION IN THE LIQUID FORM, CONTAINING AS THE ACTIVE SUBSTANCE A DRUG SUBSTANCE UNSTABLE IN AN AQUEOUS ENVIRONMENT**
SELBSTEMULGIERENDE PHARMAZEUTISCHE ZUSAMMENSETZUNG IN FLÜSSIGER FORM MIT EINER IN EINER WÄSSRIGEN UMGEBUNG INSTABILEN ARZNEIMITTELSUBSTANZ ALS WIRKSTOFF
COMPOSITION PHARMACEUTIQUE AUTO-ÉMULSIFIANTE SOUS FORME LIQUIDE, CONTENANT COMME SUBSTANCE ACTIVE UNE SUBSTANCE MÉDICAMENTEUSE INSTABLE DANS UN ENVIRONNEMENT AQUEUX

(30) Priority: 31.01.2019 PL 42877919
(43) Date of publication of application: 08.12.2021
(73) Proprietor: Gdanski Uniwersytet Medyczny, 80-210 Gdansk (PL)
(72) Inventor: SZNITOWSKA, Malgorzata, 80-772 Gdansk (PL); SYCH, Aleksandra, 62-230 Witkowo (PL); BACZEK, Tomasz, 80-174 Otomin (PL); KRZEMINSKA, Katarzyna, 80-462 Gdansk (PL)
(74) Representative: Kondrat, Mariusz
(86) International application number: PCT/IB2020/050702
(87) International publication number: WO 2020/157675

(56) References cited:
- WO-A1-2009/011861
- WO-A1-2013/012959
- FR-A1- 2 372 635
- US-A1- 2003 059 470
- US-A1- 2004 142 040
- ZAICHIK S. ET AL.: "Development and in vitro evaluation of a self-emulsifying drug delivery system (SEDDS) for oral vancomycin administration", INTERNATIONAL JOURNAL OF PHARMACEUTICS, ELSEVIER, NL, vol. 554, 5 November 2018 (2018-11-05), pages 125 - 133, XP085567942, ISSN: 0378-5173, DOI: 10.1016/J.IJPHARM.2018.11.010

## Description

The invention is a self-emulsifying pharmaceutical composition for ocular, oral, mucosal and parenteral administration, containing a drug substance unstable in an aqueous environment as set out in the appended claims.

Many drug substances cannot be produced in the liquid form ready for application because this requires appropriate stability in an aqueous solution or even in a suspension. Such substances include antibiotics. In the case of oral or parenteral formulations, powders or granules (dry formulations) for ex tempore preparation of the liquid form are then prepared. Such an approach is impractical for eye drops because combining a powder with a solvent would require an aseptic procedure, which is not possible at the patient's home. Such formulations have occasionally appeared but have not gained popularity.

Oil-based eye drops, and thus anhydrous formulations, are known. Unfortunately, oil-based solutions administered in the eye interfere with vision and are poorly tolerated by the patient. Also oral or mucosal administration of a delivery system consisting only of a hydrophobic oil is not attractive because of its taste, organoleptic sensations and poor miscibility with body fluids.

Self-emulsifying oils (SEOs) are much better tolerated, especially after oral administration, because in contact with the tear fluid they create an emulsion system, easily dispersible. Such delivery systems, which constitute a mixture of an oil with a surfactant (up to 5%) have already been tested in vitro (cellular cultures) and in vivo (rabbit eye) and their good tolerability and non-toxicity have been demonstrated [E. Wolska, M. Sznitowska, J. Chorazewicz, O. Szerkus, A. Radwahska, M.J. Markuszewski, R. Kaliszan, K. Raczyriska, Ocular irritation and cyclosporine A distribution in the eye tissues after administration of Solid Lipid Microparticles in the rabbit model, Eur. J. Pharm. Sei. 2018; vol. 121, p. 95-105. 2. A. Czajkowska-Kosnik, M. Sznitowska, K. Mirkowska, Self-emulsifying oils for ocular drug delivery. II. In vitro release of indomethacin and hydrocortisone, Acta Pol. Pharm. - Drug Res. 2012; vol. 69, No. 2, p. 309-317. Kosnik, E. Wolska, J. Chorazewicz, M. Sznitowska, Comparison of cytotoxicity in vitro and irritation in vivo for aqueous and oily solutions of surfactants. Drug Dev. Ind. Pharm. 2015; p. 1232-1236.]. The emulsion system created "in situ" is particularly attractive in a situation where eye drops in the form of emulsions have already been introduced to the clinical use as medical devices (hydrating drops) or as medicinal products (with ciclosporin).

The literature defines self-emulsifying drug delivery systems as isotropic mixtures of an oil, surfactant, with a possible addition of a co-surfactant (or solubiliser) and an active substance, which can form oil-in-water type emulsions or microemulsions as a result of a gentle mixing after dilution with an aqueous phase.

Self-emulsifying oils have already been described as delivery systems for drug substances with poor solubility in water. Owing to the presence of the oil phase and surfactants (usually at least two), good solubility of substances is obtained, also when SEO is mixed with water or body fluids. Because the drug substance can be absorbed only when it exists in the dissolved form, such systems are used for increasing the absorption (bioavailability), and as a result the efficacy of drug substances that are poorly soluble in water. Another important factor is the interaction between the surfactants contained in the SEO and biological barriers (membranes) in the body, because a common phenomenon is promotion of absorption through a change in permeability of the barrier under the effect of surfactants.

In contact with an aqueous environment, the self-emulsifying oils may form emulsions or microemulsions. Microemulsion-forming systems require careful selection of the appropriate proportions of their ingredients and an addition of a co-surfactant. Depending on this, SEDDS (self-emulsifying drug delivery systems) and SMEDDS (self-microemulsifying drug delivery systems) are distinguished. Because of higher promotion of absorption, SMEDDS raise the most interest and there are also numerous patents for those systems. Unfortunately, because of the higher concentration of surfactants and the presence of co-surfactants, SMEDDS are associated with a greater risk of irritation (for example in the eye) than self-emulsifying oils of the SEDDS type.

Self-emulsifying oils may be administered orally, on the mucosa, on the skin, parenterally, into the eye and into the ear, but because of the high risk of irritation with other routes of administration, formulations for oral use are being primarily developed, which include the use of such oils as a filling in soft capsules.

Self-emulsifying drug delivery systems that contain the oil phase and more than one surfactant are known.

Patent application IN201611003941-A applies to a self-emulsifying pharmaceutical nanocomposition containing iii) surface-active agent (surfactant); iv) co-surfactant. Additionally, in the above pharmaceutical nanocomposition the weight ratio of the above oil phase to the combination of surfactant and co-surfactant is 1 : 9 (and preferably 1 : 1). Moreover, upon contact with the gastrointestinal tract fluid, the self-emulsifying pharmaceutical nanocomposition creates spontaneously nonoparticles of a size ranging from 15 nm to 200 nm. This composition contains an additional surfactant, which is unfavourable for the patient because of an increased risk of an irritant effect upon ocular administration.

From patent application FR2372635 A1, the composition in the SMEDDS form is also known, which contains a unit dose of an active ingredient and of a self-emulsifying oil ingredient. Preferably, the active ingredients and the self-emulsifying oil ingredient are present in a solution or suspension. Furthermore, the patent application discloses a pharmaceutical composition in the form of a soft gelatine capsule containing a unit dose of the active ingredient and self-emulsifying oil ingredient consisting of an oil and non-ionic surfactant. The purpose of the composition according to patent application FR2372635 is only to increase absorption of the active substance without noting the aspect of stability of the active substance in an aqueous environment. Furthermore, the composition discussed is intended for solubilisation of water-insoluble active substances such as diazepam. A technical issue resolved by such type of delivery systems is not only dissolution of the drug substance but also prevention of its recrystallisation after mixing with water, also at the target site of action of the formulation.

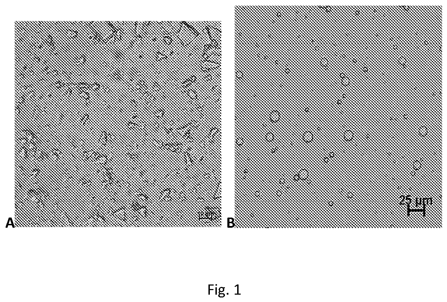

The present invention is a pharmaceutical composition in the form of a SEO -also called self-emulsifying drug delivery system (SEDDS) (Fig. 1). In the delivery system, the water-soluble drug substance forms a suspension. After application to the eye, the emulsion is rapidly formed, and the drug substance dissolves in the tear fluid, rapidly or gradually. As a result, the obtained system constitutes an aqueous solution in which oil microdroplets constitute an "additive" to the solution prolonging the time the preparation remains in contact with the eye. In contrast to the above-listed inventions, and above all to the SMEDDS concept, the product developed does not serve to increase solubility of the active substance but the solution of this substance is formed after the addition of water, without the phenomenon of increased solubility because of the presence of surfactants. The above-described process of emulsifying an anhydrous delivery system in contact with an aqueous environment, with concomitant dissolution of the drug substance, may occur also after oral or mucosal administration.

The active substance suspension in an SEO is an anhydrous system which can be stored for a long time without decomposition of the drug substance. Since the drug substance is dispersed in the form of solid particles, high chemical stability is further guaranteed. Some active substances (depending on their lipophilicity and concentration) may be dissolved in such system, but it is not necessary. Many drug substances are poorly oil-soluble and thus a suspension is more universal. Drug substances that are poorly oil-soluble are often well-soluble in the tear fluid. Additionally, a suspension offers the possibility of preparation of formulations at different concentrations, without limitation by solubility.

The purpose of the present invention is to develop a new, stable pharmaceutical composition in the liquid form, containing in particular antibiotics as the active substances, the manufacture of which is uncomplicated and is possible with the use of the standard formulation methods. The drug delivery system forms an emulsion in the tear fluid, where the drug substance rapidly dissolves, attaining a high concentration. In contrast to oil-based formulations, SEO is well tolerated by the eye, which has been proven in in vivo studies.

The invention applies above all to eye drops but it may be a drug formulation administered by other routes, on condition of contact with the aqueous environment in vivo: orally, on the mucosa, parenterally. Moreover, an emulsion can be obtained ex tempore by adding the aqueous phase before the product is administered to the patient.

It is not necessary to dissolve the drug substance in the SEO - a form of suspension is obtained but the substance can also be dissolved.

The essence of the invention is a self-emulsifying pharmaceutical composition in the liquid form, comprising a delivery system for increasing chemical stability of an active substance, comprising a delivery system for increasing chemical stability of an active substance, containing as the active substance a drug substance unstable in an aqueous environment, characterised in that the drug substance unstable in the aqueous environment is a water soluble substance which is stable in aqueous solution for less than 1 year, wherein a dissolution of said substance requires a delivery of <1 to 30 weight parts of water per 1 weight part of the dissolved substance, said composition contains the active substance in a quantity of 0.5-10 wt%, an oil in a quantity of 84-99 wt% and a surfactant in a quantity of 0.5-6 wt%, wherein the active substance is dispersed in a mixture of an oil and a surfactant; wherein said drug substance unstable in an aqueous environment is selected from a group comprising compounds belonging to the group of esters of carboxylic and inorganic acids, amides, nitriles, alkyl halogens, sulphochlorides, epoxides and carbamates, peptides, proteins, nucleic acids and wherein said composition is administered orally, into the eye, on the mucosa, or parenterally.

The present invention also covers the manufacturing method of a stable pharmaceutical composition according to the invention, in the liquid form, characterised in that this method consists of the following steps: mixing of the oil and surfactant, filtration at an elevated temperature, if necessary through a sterilising filter, suspending the drug substance (sterile if necessary) unstable in an aqueous environment, homogenisation of the suspension. Wherein said drug substance is a water soluble substance which is stable in aqueous solution for less than 1 year.

The following terms are defined below:
a) The term of self-emulsifying pharmaceutical composition according to the invention means the composition from which upon contact with water emulsion is formed and the active substance dissolves partially or completely in the aqueous phase. Additionally, the composition according to the invention does not contain water and the above-mentioned partial or complete dissolution of the active substance in the aqueous phase takes place at the site of application/action of the composition, e.g. in the eye, where the aqueous phase is the tear fluid. Additionally, the above-mentioned active substance used in the composition according to the invention is a water-soluble substance, i.e. a substance for dissolving the dissolution of which delivery of <1 to 30 weight parts of a solvent per 1 weight part of the dissolved substance is required. Dissolution of the active substance in the aqueous phase at the target site of action of the composition (e.g. in the tear fluid in the eye) depends also on time and may be complete or partial, where partial dissolution is a result of, for example, too small quantity of the tear fluid in the eye and is not related to water solubility of the given substance.
b) Oil

The oil used in the pharmaceutical composition according to the invention was selected from the group including soybean oil, MCT (medium chain triglycerides) oil, castor oil, peanut oil. The oil in the composition is a hydrophobic solvent or a mixture of such solvents which include natural oils, e.g. soybean oil, peanut oil, castor oil, sesame oil, or semi-synthetic oils, and also mineral and silicone oils. According to the invention, an oil selected from fatty acid esters with glycerol, fatty acids esters with aliphatic alcohols, unsaturated fatty acids and their mixtures. Viscosity of the oil may be modified, e.g. by isopropyl myristate or lipids with a higher melting point.

The composition may additionally contain an antioxidant, e.g. vitamin E or butylhydroxytoluene.

### c) Surfactant

Surfactants in the composition are amphiphilic compounds used in pharmaceutical formulations as emulsifiers of w/o or o/w type, consisting of hydrophilic and lipophilic chemical groups. Hydrophilic groups include hydroxyl, acid, amine, amide, sulphate, phosphate or sulphonic groups, sugars, polyhydroxy alcohols, polyoxyethylene glycols. Lipophilic groups are aliphatic hydrocarbon chains (C8 - C20), either straight or branched, or polyoxypropylene substituents, and also alkylonaphtalene residues (alkyl groups C>3), long chains of fluoroalkyl groups, polysiloxane groups. Both ingredients are linked by ester, ether or amide bonds- Surfactants can be used in the composition alone or as mixtures.

According to the invention, the surfactant should be selected from: polysorbates (fatty acid esters with polyoxyethylene sorbitan), fatty acid esters with sorbitan (e.g. Span), monoglycerides, polyoxyethylated glycerides of castor oil (e.g. Cremophor), poloxamers or their mixtures.

### d) Active substance unstable in an aqueous environment

An active substance unstable in an aqueous environment is considered any substance that decomposes in the presence of water. With regard to the invention, these are water-soluble substances which are stable in aqueous solutions for less than 1 year, which means that their content at 12 months of storage in the refrigerator or at room temperature decreases by at least 5% or the content of decomposition products exceeds 0.2%. Such substances contain ester, amide, lactam and imide groups and belong to the group of amides, nitriles, carboxylic and inorganic acid esters, alkyl halides, sulphochlorides, epoxides and carbamates. Examples of drug substances that are unstable in an aqueous environment are antibiotics and other antimicrobial drugs, vitamins, non-steroidal anti-inflammatory agents, cytostatics, peptides, proteins, nucleic acids.

The invention is illustrated by the following examples that do not limit its scope in any way and Figure 1 presenting the microscopic image of the vancomycin suspension in a mixture of the oil with surfactant (A) and after the formulation was mixed with water (B).

Polysorbate 20 means polyoxyethylene sorbitan monolaurate.

Polysorbate 80 means polyoxyethylene sorbitan monooleate

Span 80 means sorbitan monooleate.

MCT oil means medium chain triglycerides.

Cremophor EL means Polyoxyl 35 hydrogenated castor oil.

### Example 1

| | |
|---|---|
| Vancomycin | 5% (w/w) |
| Polysorbate 20 | 1% |
| Soybean oil | 94% |

The oil was mixed with Polysorbate and vancomycin was added - the latter becomes suspended. The suspension was homogenised. The suspension was dispensed into tightly closed vials.

Stability of vancomycin after 6 months of storage at room temperature was tested. The HPLC method was used for this purpose. The comparison of the chromatogram of the formulation at time t=0 and after 6 months did not demonstrate any additional product peaks resulting from vancomycin decomposition. 98-105% of the initial content of vancomycin was determined (on the basis of the peak area).

### Example 2

| | |
|---|---|
| Vancomycin | 5% |
| Cremophor EL | 1% |
| MCT oil | 94% |

The oil was mixed with the surfactant Cremophor EL and vancomycin was suspended. The suspension was homogenised and dispensed into vials. In tightly closed vials, the product was heated for 20 min in an autoclave, at 121°C.

Vancomycin stability was tested by HPLC. The comparison of the chromatogram of the formulation before heating and after autoclaving did not demonstrate any additional product peaks resulting from vancomycin decomposition. 95% of the initial content of vancomycin was determined (on the basis of the peak area), evidencing the lack of decomposition in the course of thermal sterilisation.

### Example 3

| | |
|---|---|
| Vancomycin | 1% |
| Span 80 | 1% |
| MCT oil | 98% |

The oil was mixed with the Span surfactant and vancomycin was suspended. The suspension was homogenised. The suspension was dispensed into tightly closed vials.

As reported in the literature, when temperature increases from 25°C to 40°C, the rate of active substance decomposition increases up to 8 times (Bouwman-Boer, Fenton-May, Brun L. Practical Pharmaceutics. Springer; 2009, p. 452).

Therefore, in this embodiment example, stability of vancomycin was tested after 3 months of storage at 40°C. The HPLC method was used. The comparison of the chromatogram of the formulation at time t=0 and after 3 months did not demonstrate any additional product peaks resulting from vancomycin decomposition. 96-103% of the initial content of vancomycin was determined (on the basis of the peak area).

### Example 4

| | |
|---|---|
| Vancomycin | 5% |
| Cremophor EL | 1% |
| Soybean oil | 94% |

The oil was mixed with the Cremophor surfactant, heated to 50°C and sterilised by filtration. In aseptic conditions, sterile vancomycin was suspended. The suspension was homogenised.

The suspension was mixed with water at a ratio of 1:10. A stable o/w emulsion was obtained, with an internal phase droplet size not exceeding 25 µm. Vancomycin was dissolved. The microscopic image of the vancomycin suspension in a mixture of the oil with surfactant (A) and after the formulation was mixed with water (B) is shown in Fig. 1. Figure 1A presents the 1-20 µm crystals of undissolved vancomycin suspended in the self-emulsifying oil. The self-emulsifying oil creates a uniform dispersing phase. After the addition of water, vancomycin crystals disappear because vancomycin becomes dissolved in water and the oil in the aqueous phase forms 1-15 µm droplets, creating an oil-in-water emulsion.

### Example 5

| | |
|---|---|
| Vancomycin | 5% |
| Polysorbate 80 | 5% |
| Castor oil | 90% |

The oil was mixed with the Polysorbate surfactant and vancomycin was suspended. The suspension was homogenised. The suspension was dispensed into tightly closed vials.

The suspension was mixed with water at a ratio of 1:2. A stable o/w emulsion was obtained, with an internal phase droplet size not exceeding 25 µm. Vancomycin was dissolved.

### Example 6

| | |
|---|---|
| Cefuroxime sodium | 1% |
| Polysorbate 20 | 1% |
| MCT oil | 98% |

The oil was mixed with the Polysorbate surfactant and cefuroxime was suspended. The suspension was homogenised. The suspension was dispensed into tightly closed vials.

The suspension was mixed with water at a ratio of 1:10. A stable o/w emulsion was obtained, with an internal phase droplet size not exceeding 25 µm. Cefuroxime was dissolved.

### Example 7

| | |
|---|---|
| Ascorbic acid | 5% |
| Polysorbate 80 | 1% |
| Peanut oil | 94% |

The oil was mixed with the Polysorbate surfactant and ascorbic acid was suspended. The suspension was homogenised. The suspension was encapsulated in a gelatine capsule. The capsules were stored at 40°C for 1 month. Analysis of ascorbic acid using the capillary electrophoresis method was performed. No ascorbic acid decomposition products were detected.

### Example 8 (reference example)

| | |
|---|---|
| Polysorbate 20 | 5% |
| MCT oil | 95% |

The oil was mixed with Polysorbate. The solution was sterilised by filtration. The solution was instilled in rabbit eyes 3 times (every 8-10 hours), 10 µl per eye. The appearance of the conjunctiva, cornea and iris was observed for 48 hours after the first instillation and was scored on the Draize scale. Non-significant irritant action was demonstrated only within the conjunctiva. No reaction of the cornea and iris was detected. The MAS (maximum average score) on the scale of 1-110 was 5.6.

### Example 9

| | |
|---|---|
| Vancomycin | 1% |
| Cremophor EL | 1% |
| Soybean oil | 98% |

The oil was mixed with the Cremophor EL surfactant, heated to 50°C and sterilised by filtration. In aseptic conditions, sterile vancomycin was suspended. The suspension was homogenised.

A test of antimicrobial activity was conducted by the diffusion method described in the European Pharmacopoeia. The suspension was diluted with a self-emulsifying oil to vancomycin concentration of 100 µg/ml, and then was further diluted with a buffer pH 8.0 to 20 µg/ml and 5 µg/ml. The emulsion obtained was placed on a solid agar culture medium with the test reference strains (Bacillus subtilis). The size of the microbial growth inhibition zone was examined. As a control the respective dilutions of vancomycin aqueous solution were tested. At 18 hours, the microbial growth inhibition zones after application of the tested formulation corresponded to 96% and 90% of the size of the growth inhibition zones observed in the control sample (vancomycin solution at a concentration of 20 µg/ml and 5 µg/ml, respectively).

## Claims

1. A self-emulsifying pharmaceutical composition in the liquid form, comprising a delivery system for increasing chemical stability of a water soluble active substance, a dissolution of said substance requires a delivery of <1 to 30 weight parts of water per 1 weight part of the dissolved substance, said composition contains the active substance in a quantity of 0.5-10 wt%, an oil in a quantity of 84-99 wt% and a surfactant in a quantity of 0.5-6 wt%, wherein the active substance is dispersed in a mixture of an oil and a surfactant; wherein said drug substance unstable in an aqueous environment is selected from a group consisting of antibiotics, antimicrobial drugs, vitamins, non-steroidal anti-inflammatory agents, cytostatics, peptides, proteins, nucleic acids, said surfactant forms stable emulsions and is selected from the group consisting of polysorbates, fatty acid esters with sorbitan, monoglycerides, polyoxyethylated glycerides of castor oil, poloxamers or their mixtures; said oil is selected from fatty acid esters with glycerol, fatty acids esters with aliphatic alcohols, unsaturated fatty acids and their mixtures and wherein said composition is administered orally, into the eye, on the mucosa, or parenterally.

2. A manufacturing method of a stable self-emulsifying pharmaceutical composition in the liquid form according to claim 1, **characterized in that** this method consists of the following steps:
- oil and surfactant mixing,
- filtration at an elevated temperature, if necessary, through a sterilising filter,
- suspending a water soluble active substance, wherein said active substance is selected from a group comprising antibiotics, antimicrobial drugs, vitamins, non-steroidal anti-inflammatory agents, cytostatics, peptides, proteins, nucleic acids,
- homogenisation of the suspension.

## Patentansprüche

1. Selbstemulgierende pharmazeutische Zusammensetzung in flüssiger Form, umfassend ein Abgabesystem zur Erhöhung der chemischen Stabilität einer wasserlöslichen aktiven Substanz, wobei eine Auflösung der Substanz eine Abgabe von <1 bis 30 Gewichtsteilen Wasser pro 1 Gewichtsteil der gelösten Substanz erfordert, wobei die Zusammensetzung die aktive Substanz in einer Menge von 0,5-10 Gew.-%, ein Öl in einer Menge von 84-99 Gew.-% und ein Tensid in einer Menge von 0. 5-6 Gew.-%, wobei der Wirkstoff in einer Mischung aus einem Öl und einem oberflächenaktiven Mittel dispergiert ist; wobei die in wässriger Umgebung instabile Arzneimittelsubstanz aus einer Gruppe ausgewählt ist, die aus Antibiotika, antimikrobiellen Arzneimitteln, Vitaminen, nicht-steroidalen entzündungshemmenden Mitteln, Zytostatika, Peptiden, Proteinen und Nukleinsäuren besteht, das Tensid stabile Emulsionen bildet und aus der Gruppe ausgewählt ist, die aus Polysorbaten, Fettsäureestern mit Sorbitan, Monoglyceriden, polyoxyethylierten Glyceriden von Rizinusöl, Poloxameren oder deren Mischungen besteht; das Öl ausgewählt ist aus Fettsäureestern mit Glycerin, Fettsäureestern mit aliphatischen Alkoholen, ungesättigten Fettsäuren und deren Mischungen und wobei die Zusammensetzung oral, ins Auge, auf die Schleimhaut oder parenteral verabreicht wird.

2. Verfahren zur Herstellung einer stabilen, selbstemulgierenden pharmazeutischen Zusammensetzung in flüssiger Form gemäß Anspruch 1, **dadurch gekennzeichnet, dass** dieses Verfahren aus den folgenden Schritten besteht:
- Mischen von Öl und Tensid,
- Filtration bei erhöhter Temperatur, gegebenenfalls durch ein Sterilisationsfilter,
- Suspendieren eines wasserlöslichen Wirkstoffs, wobei der Wirkstoff aus einer Gruppe ausgewählt ist, die Antibiotika, antimikrobielle Arzneimittel, Vitamine, nichtsteroidale entzündungshemmende Mittel, Zytostatika, Peptide, Proteine, Nukleinsäuren umfasst,
- Homogenisierung der Suspension.

## Revendications

1. Composition pharmaceutique auto-émulsifiante sous forme liquide, comprenant un système d'administration pour augmenter la stabilité chimique d'une substance active soluble dans l'eau, une dissolution de ladite substance nécessite une administration de <1 à 30 parties de poids d'eau pour 1 partie de poids de la substance dissoute, ladite composition contient la substance active dans une quantité de 0,5-10 wt%, une huile dans une quantité de 84-99 wt% et un tensioactif dans une quantité de 0.5-6 % en poids, la substance active étant dispersée dans un mélange d'huile et de surfactant; dans lequel ladite substance médicamenteuse instable en milieu aqueux est choisie dans un groupe constitué d'antibiotiques, de médicaments antimicrobiens, de vitamines, d'agents anti-inflammatoires non stéroïdiens, de cytostatiques, de peptides, de protéines, d'acides nucléiques, ledit surfactant forme des émulsions stables et est choisi dans le groupe constitué de polysorbates, d'esters d'acides gras avec le sorbitan, de monoglycérides, de glycérides polyoxyéthylés d'huile de ricin, de poloxamères ou de leurs mélanges ; ladite huile est choisie parmi les esters d'acides gras avec le glycérol, les esters d'acides gras avec les alcools aliphatiques, les acides gras insaturés et leurs mélanges et dans laquelle ladite composition est administrée par voie orale, dans l'œil, sur la muqueuse ou par voie parentérale.

2. Procédé de fabrication d'une composition pharmaceutique auto-émulsifiante stable sous forme liquide selon la revendication 1, **caractérisé en ce que** ce procédé consiste en les étapes suivantes :
- mélange de l'huile et du tensioactif,
- filtration à température élevée, si nécessaire, à travers un filtre stérilisant,
- mise en suspension d'une substance active soluble dans l'eau, ladite substance active étant choisie dans un groupe comprenant des antibiotiques, des médicaments antimicrobiens, des vitamines, des agents anti-inflammatoires non stéroïdiens, des cytostatiques, des peptides, des protéines, des acides nucléiques,
- homogénéisation de la suspension.
